(19) Europäisches Patentamt
European Patent Office
Office européen des brevets

(11) Veröffentlichungsnummer : **0 411 410 B1**

(12) # EUROPÄISCHE PATENTSCHRIFT

(45) Veröffentlichungstag der Patentschrift :
**30.06.93 Patentblatt 93/26**

(51) Int. Cl.$^5$ : **C07C 29/36**

(21) Anmeldenummer : **90113916.2**

(22) Anmeldetag : **20.07.90**

(54) **Verfahren zur Herstellung von Oktadienolen.**

(30) Priorität : **29.07.89 DE 3925217**

(43) Veröffentlichungstag der Anmeldung :
**06.02.91 Patentblatt 91/06**

(45) Bekanntmachung des Hinweises auf die Patenterteilung :
**30.06.93 Patentblatt 93/26**

(84) Benannte Vertragsstaaten :
**BE DE ES FR GB IT NL**

(56) Entgegenhaltungen :
**EP-A- 0 330 999**
**EP-A- 0 361 304**
**DE-A- 2 018 054**
**DE-A- 3 112 213**

(73) Patentinhaber : **BASF Aktiengesellschaft**
**Carl-Bosch-Strasse 38**
**W-6700 Ludwigshafen (DE)**

(72) Erfinder : **Thome, Alfred, Dr.**
**Brechlochstrasse 9**
**W-6700 Ludwigshafen (DE)**
Erfinder : **Betleff, Werner, Dr.**
**Franz-Mark-Strasse 12**
**W-6806 Viernheim (DE)**

EP 0 411 410 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein verbessertes Verfahren zur Herstellung von Octadienolen durch Umsetzung von 1,3-Butadien mit Wasser in Gegenwart eines Katalysatorsystems bestehend aus einer Palladiumverbindung einer Triorganophosphorverbindung und Kohlendioxid unter Mitverwendung eines Triorganophosphinoxides.

Octadienole sind unter anderem als Zwischenprodukte zur Herstellung von Octylalkoholen verwendbar, die ihrerseits zur Herstellung von Weichmachern wie Dioctylphthalat dienen. Da hierbei das Octan-1-ol bevorzugt wird, haben solche Octadienole die größte Bedeutung, die sich wie Octa-2,7-dien-1-ol in Octan-1-ol überführen lassen, jedoch sind gerade diese Octadienole bisher nur in unzureichenden Ausbeuten erhältlich.

Aus der DE-A-20 18 054 ist es bekannt, daß die Telomerisation von Butadien mit Wasser in Gegenwart von Kohlendioxid, eines Lösungsmittels sowie eines Katalysatorsystems aus einer Palladium(O)- oder (II)-Verbindung und eines tertiären Phosphins oder Phosphits Octadienole ergibt

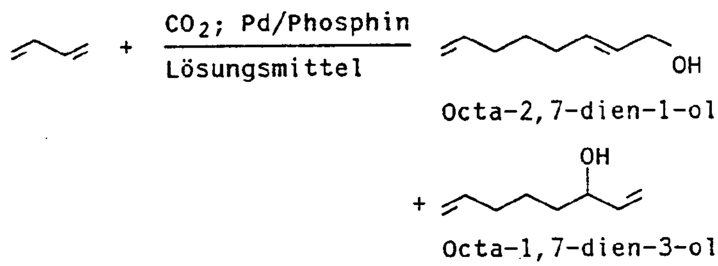


Octa-2,7-dien-1-ol

Octa-1,7-dien-3-ol

wobei man Octadienylether und Polyene wie Octa-1,3,7-trien als Nebenprodukte erhält.

Die hierbei erzielbaren Ausbeuten an den Octadienolen sowie auch die Selektivität bezüglich des Octa-2,7-dien-1-ols sind jedoch unbefriedigend.

In der EP-A-330 999 wird ein kontinuierliches Verfahren beschrieben, in dem zur Verbesserung von Reaktivität und Selektivität zusätzlich zur Reaktionsmischung, bestehend aus Butadien, Wasser, Kohlendioxid, Palladiumkatalysator, Phosphinliganden und inertem Lösungsmittel, eine starke, nicht koordinierende Säure wie z. B. Tetrafluorborsäure, Hexafluorphosphorsäure oder Trifluormethansulfonsäure zugesetzt wird.

Trotzdem läßt die Raum-Zeit-Ausbeute dieser Reaktion und damit die Reaktionsgeschwindigkeit zu wünschen übrig.

DE-A-31 12 213 betrifft ein Verfahren zur Herstellung von n-Octadienol aus Butadien und Wasser mit Hilfe von Phosphin-modifizierten Palladiumkatalysatoren. Es wird darin erwähnt, daß die verwendeten Phosphine dazu neigen, von im Reaktionssystem enthaltenem Sauerstoff zu Phosphinoxiden oxidiert zu werden und dabei ihre Aktivität verlieren, weshalb in dieser Schrift Maßnahmen zur Vermeidung dieser Oxidationsreaktion vorgeschlagen werden.

Der Erfindung lag daher die Aufgabe zugrunde, die geschilderten Nachteile zu beheben und vor allem das Wertprodukt 2,7-Octadien-1-ol in höheren Raum-Zeit-Ausbeuten als bisher zugänglich zu machen.

Demgemäß wurde ein verbessertes Verfahren zur Herstellung von Octadienolen durch Umsetzung von 1,3-butadien mit Wasser in Gegenwart eines Katalysatorsystems, bestehend aus einer Palladiumverbindung, einer Triorganophosphorverbindung und Kohlendioxid gefunden, welches dadurch gekennzeichnet ist, daß man die Reaktion in Gegenwart eines Triorganophosphinoxides vornimmt, wobei das Molverhältnis Triorganophosphinoxid zu Palladium 0,1 : 1 bis 100 : 1 beträgt.

Das Triorganophosphinoxid kann dem System als Phosphinoxid zugesetzt oder durch Zugabe eines Oxidationsmittels wie z.b. Luftsauerstoff, zur Reaktionslösung aus dem Triorganophosphinligand ganz oder teilweise in das entsprechende Phosphinoxid überführt werden.

Als Organophosphinoxid-Verbindungen eignen sich prinzipiell alle lipophilen und hydrophilen Phosphinoxide. Die Menge des Organo-phosphinoxids ist nicht kritisch. Vorzugsweise kann das Molverhältnis zu Palladium 0,1 : 1 bis 100 : 1 betragen, besonders bevorzugt 0,5:1 bis 10:1. Im allgemeinen werden als Triorganophosphinoxid-Verbindungen Triaryl-, Diarylalkyl-, Aryldialkyl- sowie Trialkylphosphinoxide verwendet. Als Beispiele seien genannt: Tributylphosphinoxid, Dimethyl-n-octylphosphinoxid, Tricyclohexylphosphinoxid, Triphenylphosphinoxid, Tritolylphosphinoxid, Tris-(p-methoxyphenyl)phosphinoxid, Diphenylethylphosphinoxid, Dimethylphenylphosphinoxid. Trialkylphosphinoxide sind im allgemeinen weniger gut geeignet. besonders bevorzugte Organophosphinoxide sind, nicht zuletzt aus wirtschaftlichen Gründen, Triarylphosphinoxide wie Triphenyl- oder Tritolylphosphinoxide oder Aryl-alkylphosphinoxide wie Diphenyl-$C_1$-$C_8$-Alkylphosphinoxide. Aus wirtschaftlichen überlegungen empfiehlt es sich ferner, das Oxid des ebenfalls mitverwendeten Triorganophosphins einzusetzen.

Zur Bereitung des Katalysatorsystems eignen sich grundsätzlich alle löslichen Palladium(0)- und Palladium(II)-Verbindungen. Halogenhaltige Komplexe und Salze eignen sich weniger. Als Palladium(II)-Verbindungen kommen beispielsweise:

$Pd(OAc)_2$,
$Pd(dba)_2$ (dba = Dibenzylidenaceton),
$[Pd(acac)(PPh_3)_2]BF_4$ (acac = Acetylaceton),
$[Pd(h^3\text{-}C_3H_5(COD)]BF_4$ (COD = 1,5-Cyclooctadien) und
$[Pd(acac)(COD)]BF_4$
sowie in erster Linie Palladium-(II)-acetylacetonat in Betracht.

Als Palladium(0)-Verbindungen seien beispielsweise genannt:
Tetrakis(triphenylphosphin)palladium
Tetrakis(dimethylphenylphosphin)palladium
Tetrakis(tris-p-methoxyphosphin)palladium
Bis(triphenylphosphin)($h^2$-ethylen)palladium

Die Menge der Palladiumverbindungen ist nicht kritisch, beträgt jedoch vorzugsweise $10^{-5}$ bis $10^{-1}$, besonders $10^{-4}$ bis $10^{-2}$ Mol Palladium pro Mol Butadien. Sie geht lediglich in die Reaktionsgeschwindigkeit ein. Die eingesetzte Menge wird dabei nur durch wirtschaftliche Faktoren begrenzt.

Die tertiären Phosphorverbindungen fungieren als stabilisierende Liganden L in den aktiven Palladiumkomplexen des Typs

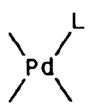 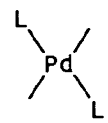 und 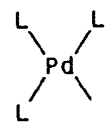

Als Liganden L eignen sich prinzipiell alle Phosphine und Phosphite, z. B. Trialkyl- und Triarylphosphine und -phosphite mit etwa bis zu insgesamt 24 C-Atomen in den Kohlenstoffresten. Als Beispiele seien genannt: Tributylphosphin, Dimethyl-n-octylphosphin, Tricyclohexylphosphin, Tritolylphosphin, Tris(p-methoxyphenyl)phosphin, Diphenylethylphosphin, Dimethylphenylphosphin, 1,2-Bis-diphenylphosphinoethan, Triethylphosphit, Tricyclohexylphosphit, Triphenylphosphit. Auch hydrophile Arylphosphine können eingesetzt werden, vorzugsweise wasserlösliche Salze von mono-, di- oder trisulfonierten Triphenylphosphinverbindungen. Besonders bevorzugt, nicht zuletzt aus wirtschaftlichen Gründen, ist das Triphenylphosphin. Allgemein ist den Phosphinen der Vorzug vor den Phosphiten zu geben, weil letztere mit dem Wasser hydrolysieren und Umlagerungsreaktionen eingehen können. Vorteilhaft, aber nicht unbedingt erforderlich, ist die Zugabe desjenigen Phosphins, welches dem erfindungsgemäßen Zusatz des Triorganophosphinoxids entspricht.

Die Menge dieser Liganden beträgt im allgemeinen 1 bis 20, vorzugsweise 1 bis 5 Mol pro Mol Palladium.

Die Menge des Kohlendioxids, welches die Butadien-Telomerisation auf bis-her nicht bekannte Weise begünstigt, ist ebenfalls nicht kritisch und kann etwa $10^{-3}$ bis 1, vorzugsweise $10^{-2}$ bis 0,5 Mol pro Mol Butadien betragen.

Für einen vollständigen Umsatz des Butadiens zu den Octadienolen sind mindestens äquimolare Mengen Wasser (d. h. Molverhältnis von Wasser zu Butadien = 0,5 : 1) erforderlich, jedoch empfiehlt sich ein höheres Molverhältnis von Wasser zu Butadien bis zu etwa 10 : 1, vorzugsweise 5 : 1, um die Konkurrenzreaktionen der Bildung von Octadienylethern, Octatrien und höheren Polyenen zu unterdrücken.

Als aprotische polare Lösungsmittel kommen vor allem Ether in Betracht, da diese sich unter den Reaktionsbedingungen inert verhalten und ein hinreichendes bis gutes Lösevermögen sowohl für Butadien als auch für Wasser aufweisen. Genannt seien Diethylether, Tetrahydrofuran und 1,4-Dioxan.

Verfahrenstechnisch besonders empfehlenswert sind solche Lösungsmittel, die höher als die gebildeten Octadienole sieden, weil man in diesem Fall die Octadienole einfach von dem katalysatorhaltigen Lösungsmittel abdestillieren kann. Geeigente hochsiedende Lösungsmittel sind vor allem Polyalkylenglykolether der Formel

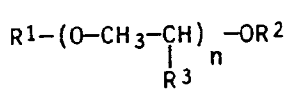

in der $R^1$ und $R^2$ $C_1$-$C_3$ Alkylgruppen und $R^3$ Wasserstoff oder die Methylgruppe bedeuten und n für einen Wert von 2 bis 6, vorzugsweise 4 steht.

Weitere geeignete hochsiedende Lösungsmittel sind Dialkylsulfoxide wie vor allem Dimethylsulfoxid und Sulfone wie Tetrahydrothiophen-1,1-di-oxid (Sulfolan).

Mit Hilfe der hochsiedenden Lösungsmittel läßt sich das erfindungsgemäße Verfahren besonders vorteilhaft kontinuierlich gestalten, indem man die genannten Komponenten in einem Reaktionsgefäß miteinander umsetzt, das Reaktionsgemisch in eine Destillationsapparatur überführt und dort nicht umgesetztes Butadien sowie das katalysatorhaltige Sumpfprodukt in das Reaktionsgefäß zurückführt.

Gegebenenfalls können auch nicht koordinierende starke Säuren verwendet werden. Hier kommen vor allem Tetrafluorborsäure, Hexafluorphosphorsäure, Methansulfonsäure, Trifluormethansulfonsäure, Schwefelsäure, Trifluoressigsäure, Trichloressigsäure und p-Toluolsulfonsäure in Betracht. Auch Fettsäuren sind nicht koordinierend, so daß sich beispielsweise auch Essigsäure eignet, die im vorliegenden Zusammenhang noch als starke Säure gilt. Schwächere Säuren als Essigsäure wird man im allgemeinen jedoch nicht verwenden, da die erfindungsgemäße Wirkung der Säuren mit abnehmender Stärke im allgemeinen geringer wird.

Unter nicht-koordinierenden Säuren sind solche zu verstehen, deren Anionen mit Übergangsmetallkationen wie Palladium keine stabilen Komplexbindungen eingehen. Näheres zur Theorie der nicht-koordinierenden Säuren ist dem Lehrbuch von F.A. Cotton und G. Wilkinson, Anorganische Chemie, 3. Auflage, Verlag Chemie, Interscience Publishers, Seiten 179, 238, 394 und 506 zu entnehmen.

Das Verhältnis von Säuren zu Palladium beträgt im allgemeinen 0,1 bis 150, vorzugsweise 50 bis 100 Äquivalent-% pro Mol Palladium.

Man nimmt die Reaktion vorzugsweise bei 50 bis 70°C vor. Unterhalb von 50°C, besonders unterhalb von 30°C, verlangsamt sich die Umsetzung zu sehr, und oberhalb von 90°C, besonders 100°C, ist mit unerwünschten Nebenreaktionen zu rechnen, die man möglicherweise dann in Kauf nehmen wird, wenn man auf hohe Raum-Zeit-Ausbeuten großen Wert legt.

Für den Druck empfiehlt sich der Eigendruck bei den gewählten Reaktionstemperaturen; er liegt meistens im Bereich von 5 bis 50, besonders 10 bis 30 bar.

Eine wirtschaftlich sehr vorteilhafte Ausführungsform des erfindungsgemäßen Verfahrens besteht in der Verwendung von sog. $C_4$-Schnitten anstelle von reinem Butadien. Die im $C_4$-Schnitt neben Butadien enthaltenen Olefine But-1-en, But-2-en und Isobuten nehmen weder an der Reaktion teil, noch beeinträchtigen sie diese. Die $C_4$-Schnitte enthalten etwa 45 Gew.-% Butadien, 17 Gew.-% But-1-en, 10 Gew.-% But-2-en, 25 Gew.-% Isobuten und den Rest Butan und Isobutan.

Das gleiche gilt für die Aufarbeitung des Reaktionsgemisches. Das Reaktionsgemisch wird aus dem Reaktor in eine Destillationsapparatur überführt. Dort werden nicht umgesetztes 1,3-Butadien sowie die Octadienole fraktioniert als Destillate abgetrennt. Anschließend werden Butadien und das katalysatorhaltige Sumpfprodukt in das Reaktionsgefäß zurückgeführt.

## Beispiele

### Beispiel 1 und Vergleichsbeispiel 1

In einem 300-ml-Autoklaven wurden unter Argon als Schutzgas 0,22 g (0,72 mMol) Palladiumacetylacetonat, Triphenylphosphin (Menge s. Tabelle 1), Triphenylphosphinoxid (Menge s. Tabelle 1) in 120 g (0,54 Mol) Tetraglyme gelöst und 0,03 g (0,34 Mol) Tetrafluorborwasserstoffsäure in 30 g (1,67 Mol) Wasser zugesetzt. Der Autoklav wurde verschlossen und 23,4 g (0,43 Mol) 1,3-Butadien sowie 4,4 g (0,1 Mol) Kohlendioxid über Druckvorlagen zugepreßt. Danach wurde die Mischung für 3 h unter Rühren auf 60°C gehalten. Nach Versuchsende wurde der Reaktor auf Raumtemperatur gebracht und entspannt. Der flüssige, leicht gelbgefärbte, einphasige Reaktoraustrag wurde gaschromatographisch analysiert. Als interner Standard wurde n-Octanol verwendet.

Tabelle 1: Autoklavenversuche zur Butadientelomerisation

| Vers.-Nr. | TPP[a) ] [g] | TPPO[b)] [g] | Ausbeute 2,7-Od-1-ol[c)] | [Mol-%] 1,7-Od-3-ol[d)] | Gesamt | n-Anteil [%] |
|---|---|---|---|---|---|---|
| Vgl.-Bsp. 1 | 0,56 | 0 | 59,8 | 5,2 | 65,0 | 92,0 |
| Bsp. 1 | 0,56 | 0,59 | 64,2 | 4,7 | 68,9 | 93,2 |

a) Triphenylphosphin b) Triphenylphosphinoxid
c) 2,7-Octadien-1-ol d) 1,7-Octadien-3-ol

Wie ein Vergleich der beiden Versuche zeigt, wurde durch den erfindungsgemäßen zusatz von Triphenylphosphinoxid, äquimolar zu Triphenylphosphin und im Molverhältnis 3 : 1 zu Palladium die Ausbeute an 2,7-Octadien-1-ol gegenüber dem Vergleichsversuch gesteigert.

Beispiele 2 und 3 und Vergleichsbeispiel 2

Zu einer in einem 2,5-l-Hubrührautoklaven befindlichen Mischung aus 1 133 g (5,1 Mol) Tetraglyme, 1,58 g (6,5 mMol) Palladium-(II)- acetylacetonat, Triphenylphosphin (Menge s. Tabelle 2), Triphenylphosphinoxid (Menge s. Tabelle 2) und 270 g (15 Mol) Wasser wurden unter einer Argon-Schutzgasatmosphäre 200 g (3,7 Mol) 1,3-Butadien zugepumpt und die Reaktionslösung auf 60°C aufgeheizt. Abschließend wurde der Kokatalysator Kohlendioxid bis zu dem gewünschten Enddruck von 10 bzw. 20 bar aufgepreßt.

Durch die Konstruktion einer reaktornahen Probenahmeeinrichtung mit der Möglichkeit, kleine Probemengen zu entnehmen, konnten pro Versuchsfahrt in kurzen Zeitintervallen mehrere Proben genommen werden.

Die Ergebnisse der Versuche bei unterschiedlichen $CO_2$-Drücken sind zusammen mit einem Vergleichsbeispiel in der Figur graphisch dargestellt und in Tabelle 2 aufgelistet.

Tabelle 2: Versuche zu Butadientelomerisation mit Wasser

| Zeit [min] | Ausbeute[a] [Mol-%] Beispiel 2[b] | Beispiel 3[c] | Vergleichsbeispiel 2[d] |
|---|---|---|---|
| 5 | 22,0 | 21,8 | 2,4 |
| 10 | 22,8 | 23,2 | 2,5 |
| 20 | 24,4 | 23,4 | 5,8 |
| 40 | 27,3 | 26,5 | 9,8 |
| 60 | 30,6 | | 13,8 |

a) Gesamtausbeute: 2,7-Octadien-1-ol und 1,7-Octadien-3-ol

b) Druck: 20 bar; 3,36 g (12,8 mmol) Triphenylphosphin und 1,78 g (6,4 mmol) Triphenylphosphinoxid

c) Druck: 10 bar; 3,36 g (12,8 mmol) Triphenylphosphin und 1,78 g (6,4 mmol) Triphenylphosphinoxid

d) Druck: 20 bar; 3,04 g (19,2 mmol) Triphenylphosphin

Die Versuche zeigen, daß bei verschiedenen Reaktionsdrücken (10 bzw. 20 bar) der Zusatz von Triphenylphosphinoxid (Molverhältnis 1 : 2 bezüglich Triphenylphosphin und 1 : 1 bzgl. Palladium) zu höheren Raum-Zeit-Ausbeuten an Octadienolen führt. Bei einem Druck von 20 bar wird mit Triphenylphosphinoxid nach 60 min Reaktionsdauer eine Gesamtausbeute an 2,7-Octadien-1-ol und 1,7-Octadien-3-ol von 30,6 Mol-% gegenüber 13,8 Mol-% ohne Oxidzusatz erreicht bei einem gleichzeitigen n-Anteil von 93,5 % gegenüber 78,9 %. Die Ergebnisse der Versuchsfahrt bei einem Druck von 10 bar sind mit denen bei 20 bar vergleichbar.

## Patentansprüche

1. Verfahren zur Herstellung von Octadienolen durch Umsetzung von 1,3-Butadien mit Wasser in Gegenwart eines Katalysatorsystems, bestehend aus einer Palladiumverbindung, einer Triorganophosphorverbindung und Kohlendioxid, dadurch gekennzeichnet, daß man die Reaktion in Gegenwart eines Triorganophosphinoxides vornimmt, wobei das Holverhältnis Triorganophosphinoxid zu Palladium 0,1 : 1 bis 100 : 1 beträgt.

## Claims

1. A process for preparing an octadienol by reacting 1,3-butadiene with water in the presence of a catalyst system comprising a palladium compound, a triorganophosphorus compound and carbon dioxide, which comprises performing the reaction in the presence of a triorganophosphine oxide in a molar ratio of triorganophosphine oxide to palladium of from 0.1 : 1 to 100 : 1.

## Revendications

1. Procédé de préparation d' octadiénols par la réaction du 1,3-butadiène avec l'eau en présence d'un système catalytique, constitué d'un composé du palladium, d'un composé triorganophosphoré et de dioxyde de carbone, caractérisé en ce que l'on entreprend la réaction en présence d'un oxyde de triorganophosphine, où le rapport molaire oxyde de triorganophosphine à palladium varie de 0,1 à 100:1.

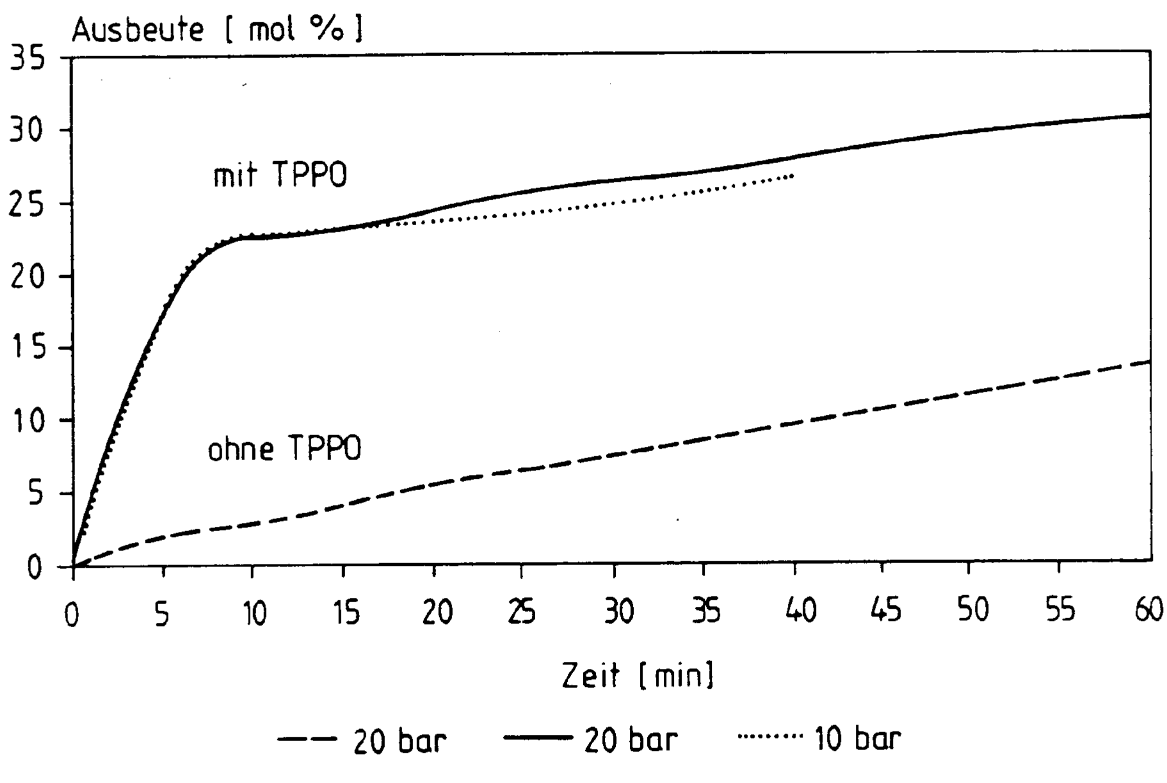

Ausbeute [ mol % ]
35
30
25
20
15
10
5
0
mit TPPO
ohne TPPO
0
5
10
15
20
25
30
35
40
45
50
55
60
Zeit [min]
20 bar
20 bar
10 bar